# EUROPEAN PATENT APPLICATION

(11) **EP 1 953 139 A1**
(43) Date of publication of application: **06.08.2008**
(21) Application number: 06833190.9
(22) Date of filing: 24.11.2006
(51) Int. Cl.: C07C 381/12

(54) **METHOD FOR PRODUCING FLUORINATED SULFONIUM ALKYLFLUOROPHOSPHATE**

(30) Priority: 25.11.2005 JP 2005341048
(71) Applicant: San-Apro Ltd., Kyoto-shi, Kyoto 605-0995 (JP)
(72) Inventor: KIMURA, Hideki, Kyoto-shi, Kyoto 605-0995 (JP); YAMAMOTO, Jiro, Kyoto-shi, Kyoto 605-0995 (JP); YAMASHITA, Shinji, Kyoto-shi, Kyoto 605-0995 (JP)
(74) Representative: Behnisch, Werner
(86) International application number: PCT/JP2006/323386
(87) International publication number: WO 2007/061024

(57) **Abstract**

Disclosed is a low cost, efficient method for production of a salt composed of an arylsulfonium and a fluorinated alkylfluorophosphate which method does not required a large excess amount of acid. The method comprises reacting an aryl compound Ar-H and with a compound represented by the formula (I), 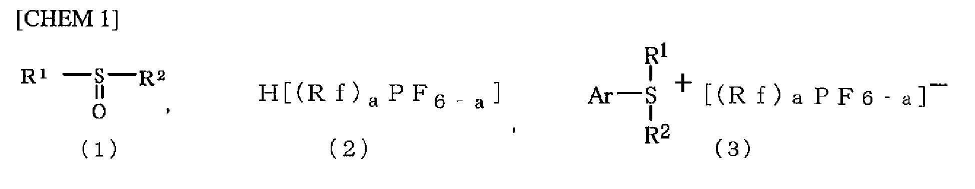 wherein R¹ and R² denote a hydrocarbon group or a heterocycle group which may be substituted, or they are bonded with each other directly or via -O-, -S-, -SO-, -SO₂-, -NH-, -NR' -, -CO-, -COO-, -CONH-, an alkylene group having 1 to 3 carbon atoms or a phenylene group to form a ring structure which may be substituted, wherein R' denotes a C1-5 alkyl group or a C6-10 aryl group; in the presence of an acid represented by the formula (2), wherein Rf denotes an alkyl group 80 % or more of whose hydrogen atoms are substituted by fluorine atoms, "a" is an integer of 1 to 5; and a dehydrating agent to produce the salt of sulfonium represented by the formula (3).

## Description

### TECHNICAL FIELD

The present invention relates to a method for producing a novel salt of a sulfonium, esp., an aryl group (aromatic ring)-carrying sulfonium, with a fluorinated alkylfluorophosphate, which is useful as a cationic photoinitiator and a photoacid generator for resists. More specifically, the present invention relates to a novel method for producing a desired sulfonium salt in high yields while reducing the production of waste liquid.

### BACKGROUND ART

As cationic photoinitiators and photoacid generators for resists, which produce acid in response to their exposure to heat or active energy radiation such as light or electron beam, there are so far known salts which include as a cation component an onium like iodonium, sulfonium, or a complex of a transition metal. Of the cation components of those salts, those which contain a sulfonium, in particular an aryl group (aromatic ring)-carrying sulfonium, are preferably used because of their high ability to initiate cationic polymerization, good storage stability of compositions consisting them together and other components like cationically polymerizable monomers, and because they will not give deep color to cured materials.

On the other hand, BF₄-, PF₆⁻, AsF₆⁻, and SbF₆⁻ are known as anionic components of those salts. The ability to initiate cationic polymerization differs among the anions, increasing in the order of BF₄ < PF₆⁻ < AsF₆⁻ < SbF₆⁻. Regarding As- and Sb-based initiators, which have potent abilities to induce photopolymerization, however, because of the disadvantage of toxicity of these metals, As-based initiators have not been put into practical use, and those based on Sb have only limited uses. Thus, PF₆⁻ salts, though inferior in the ability to initiate polymerization, are commonly used as cationic photoinitiators. However, since the ability of PF₆⁻ salts to initiate photo-induced cationic polymerization is only about one tenth of that of SbF₆⁻ salts, the amount of PF₆⁻ salts to be included in a cationic polymerization composition must be the higher for achieving a satisfactory curing rate. Thus, the employment of PF₆⁻ salts entails problems such as impairment of physical properties, and adhesiveness to a substrate, of the cured material thus obtained due to the solvent employed for, or photodecomposed products of, the initiator remaining in the cured material. For this reason, there has been a great need for such a cationic initiator that is free of toxic metals like Sb and As, has high ability to initiate cationic polymerization, is miscible with cationic polymerization composition, and yet endows good stability to the composition consisting of it and cationic polymerization monomers. To address this need, the present applicants have proposed in Japanese patent application No. 2004-159921 (not yet published at the time of the filing of the present application) salts of such oniums as sulfonium or iodonium, or of transition metal complexes, with a fluorinated alkylfluorophosphate anion.

Sulfonium salts based on such an anion is obtained by a method in which, starting from an aryl compound and a sulfoxide, a sulfonium is first prepared in the form of a salt such as a salt with an halogen ion like F⁻, Cl⁻, Br⁻ , I⁻; a salt with OH⁻; a salt with ClO₄⁻; a salt with a sulfonate like FSO₃⁻, ClSO₃⁻, CH₃SO₃⁻, C₆H₆SO₃⁻, or CF₃SO₃⁻; a salt with sulfate or a like ion such as HSO₄⁻, and SO₄²-; a salt with carbonate or a like ion such as HCO₃-, and CO₃²-; a salt with phosphate or a like ion such as H₂PO₄⁻; HPO₄²- ; and PO₄³⁻; a salt with an ion of a polyhalo metal or polyhalo metalloid represented by the general formula MXₘYₙ [wherein M denotes an element of the group IIIa or Va in the periodic table of the elements, X denotes a halogen, and Y denotes hydroxyl group, respectively, and, with respect to m and n, m + n = 4 and n is an integer 0 to 3 if M belongs the group IIIa, and m + n = 6 and n is an integer of 0 to 2 if M belongs to the group Va], and then adding the salt thus obtained to an aqueous solution of a salt of fluorinated alkylfluorophosphoric acid with an alkali metal, an alkaline earth metal, which is represented by M'[(Rf)aPF6-a] (M' denotes an alkali metal or a alkaline earth metal) or a quaternary ammonium thereof, and letting a double decomposition reaction take place.

The above method is very time-consuming to obtain the final, aimed product. In the case, in particular, of a sulfonium salts having one or more aryl groups, a large excess amount of acid and acid anhydride is required in the step of production of their sulfate, hydrogen sulfate, methanesulfonate or the like (see, for example, Patent Documents 1, 2, and 3), and, further, a large amount of water is needed to prepare an aqueous solution of the salt of the fluorinated alkylfluorophosphate with an alkali or alkaline earth metal, which is used in a subsequent process, because the concentration of the salt in the solution must be set at a low level considering the low water solubility of the salt and precipitation of inorganic salts formed as by-products. As a result, the method has a disadvantage that it leaves a large amount of waste liquid after the recovery of the aimed product. Moreover, this waste liquid is strongly acidic because it contains acid such as sulfuric acid or methanesulfonic acid and acid anhydride used in a large excess amount in the step of production of sulfonium sulfate, bisulfate or methanesulfate or the like. Therefore, the waste liquid must be neutralized with, e.g., sodium hydroxide before disposition of it, and this leads to an additional disadvantage of further increase in the amount of the waste liquid.

Furthermore, there are other disadvantages, e.g., lowered yields of the aimed product due to the sulfonation of the aryl groups of the starting aryl compound, or of the final product, which will take place where a large amount of sulfonic acid is used in the sulfonium salt-producing reaction (see Patent Document 1), or elevated costs where a costly alkylsulfonic acid such as methanesulfonic acid is employed (see Patent Document 3).

Another method for production of a sulfonium also has been proposed which is effective where, in the reaction employed to prepare the sulfonium from a sulfoxide and a sulfide, the anion part of the sulfonium salt is a polyhalo metal or a polyhalo metalloid such as BF₄⁻, PF₆⁻ , AsF₆⁻ or SbF₆⁻ (see Patent Document 4). However, as the raw materials for the production of such anions are costly, the method is not free of a disadvantage that it renders the final product costly.
[Patent Document 1] Japanese Patent Application Publication No. S61-212554
[Patent Document 2] Japanese Patent Application Publication No. S61-100557
[Patent Document 3] Japanese Patent Application Publication No. H7-82244
[Patent Document 4] Japanese Patent Application Publication No. 2002-241363

### DISCLOSURE OF INVENTION

### PROBLEMS TO BE SOLVED BY THE INVENTION

It is the objective of the present invention to provide a method for production of a salt of a sulfonium, esp. an aryl group-carrying sulfonium with a fluorinated alkylfluorophosphate, which is free of highly toxic elements such as As or Sb and exhibits excellent performances as cationic photoinitiator and a photoacid generator, wherein the method allows to directly produce the aimed compound without relying on the use of a large excess amount of acid, and is less costly and is efficient.

### MEANS TO SOLVE THE PROBLEM

As a result of studies addressed to the above problems, the inventors of the present invention found a method, in which an aryl compound and a sulfoxide compound are let undergo dehydration condensation reaction in the presence of a fluorinated alkylfluorophosphate and a dehydrating agent, and have completed the present invention through additional studies. Thus, the present invention provides what follows.

(1) A method for production of a salt of sulfonium having as a counter ion a fluorinated alkylfluorophosphate anion, which method comprises reacting an aryl compound Ar-H (A) having a hydrogen atom bonded to at least one of the carbon atoms thereof with a sulfoxide compound (B) represented by the formula (I),

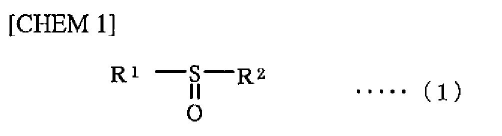

wherein R¹ and R² are the same or different from each other, and each of them denotes a hydrocarbon group which may be substituted or a heterocycle group which may be substituted, or they are bonded with each other directly or via -O-, -S-, -SO-, -SO₂-, -NH-, -NR' -, -CO-, -COO-, -CONH-, an alkylene group having 1 to 3 carbon atoms or a phenylene group to form a ring structure which may be substituted, wherein R¹ denotes an alkyl group having 1 to 5 carbon atoms or an aryl group having 6 to 10 carbon atoms; in the presence of a fluorinated alkylfluorophosphoric acid (C) represented by the formula (2),

[CHEM 2] H[(Rf)ₐPF₆₋ₐ] (2)

wherein Rf denotes an alkyl group 80 % or more of whose hydrogen atoms are substituted by fluorine atoms, "a" is an integer of 1 to 5 and indicates the number of Rf, wherein Rf occurring "a" times may be identical with or different from one another; and a dehydrating agent (D) to produce the salt of sulfonium having as a counter ion a fluorinated alkylfluorophosphate anion, wherein the salt is represented by the formula (3),

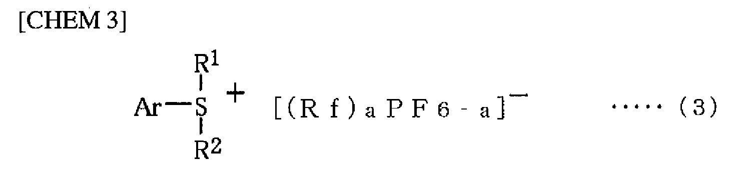

wherein R¹ and R² are as defined above, Ar denotes an aryl group derived by elimination of the hydrogen atom from the aryl compound Ar-H (A) having a hydrogen atom bonded to at least one of the carbon atoms thereof, and Rf and "a" are as defined above.
(2) The method for production according to (1) above, wherein the aryl compound (A) has at least one arylthio group which may be substituted.
(3) The method for production according to (1) or (2) above, wherein R¹ and R² in the formula (1) is a phenyl group which may be substituted.
(4) The method for production according to one of (1) to (3) above, wherein the fluorinated alkylfluorophosphoric acid (C) is selected from the group consisting of H[(CF₃CF₂)₃PF₃], H[(CF₃CF₂CF₂)₃PF₃], H[((CF₃)₂CFCF₂)₃PF₃], and H[((CF₃)₂CFCF₂)₂PF₄].
(5) The method according to one of (1) to (4) above, wherein the dehydrating agent (D) is acetic anhydride.
(6) The method according to one of (1) to (5) above, wherein the fluorinated alkylfluorophosphoric acid (C) is formed in a reaction system comprising at least any one of the aryl compound (A), the sulfoxide compound (B), the dehydrating agent (D), and a solvent by adding to the reaction system a salt of fluorinated alkylfluorophosphoric acid with an alkali metal or an alkaline earth metal, and sulfuric acid.
(7) The method according to (6) above, wherein the salt of the fluorinated alkylfluorophosphoric acid is a salt of the fluorinated alkylfluorophosphoric acid with at least one alkali metal selected from the group consisting of Li, K, and Na.

### EFFECT OF THE INVENTION

The present invention enables to produce a salt of sulfonium, esp., an aryl group-carrying sulfonium, with a fluorinated alkylfluorophosphate, which salt is free of highly toxic elements such as As or Sb and exhibits excellent performances as a cationic photoinitiator and a photoacid generator, without using a large excess amount of acid, and at low cost and with high efficiency.

### BEST MODE FOR CARRYING OUT THE INVENTION

In the present invention, examples of an aryl compound (A) having a hydrogen atom bonded to at least one of carbon atoms thereof (abbreviated as "Ar-H") include monocyclic aromatic hydrocarbons such as benzene or condensed polycyclic aromatic hydrocarbons such as or naphthalene, anthracene, phenanthrene, pyrene, chrysene, naphthacene, benzanthracene, anthraquinone, fluorene, and naphthoquinone, having 6 to 30 carbon atoms, as well as heterocyclic compounds having 1 to 3 hetero atoms such as oxygen, nitrogen, sulfur and the like, wherein such hetero atoms may be the same or different from each other, such as monocyclic heterocyclic compound like thiophene, furan, pyrrole, oxazole, thiazole, pyridine, pyrimidine, pyrazine, and condensed polycyclic heterocyclic compounds such as indole, benzofuran, benzothiophene, quinoline, isoquinoline, quinoxaline, quinazoline, carbazole, acridine, phenothiazine, phenazine, xanthene, thianthrene, phenoxazine, phenoxathiine, chroman, isochroman, dibenzothiophene, xanthone, thioxanthone, dibenzofuran, and the like, having 4 to 30 carbon atoms.

The above mentioned aromatic hydrocarbons having 6 to 30 carbon atoms or the heterocyclic compounds having 4 to 30 carbon atoms may be substituted by one or more substituents selected from the group consisting of alkyl, hydroxyl, alkoxyl, alkylcarbonyl, arylcarbonyl, alkoxycarbonyl, aryloxycarbonyl, arylthiocarbonyl, acyloxy, arylthio, alkylthio, aryl, a hetero ring, aryloxy, alkylsulfinyl, arylsulfinyl, alkylsulfonyl, arylsulfonyl, alkyleneoxy, amino, cyano, nitro, and halogen.

Concrete examples of the above substituents include straight-chain alkyl groups having 1 to 18 carbon atoms such as methyl, ethyl, propyl, butyl, pentyl, octyl, decyl, dodecyl, tetradecyl, hexadecyl, and octadecyl; branched alkyl groups having 1 to 18 carton atoms such as isopropyl, isobutyl, sec-butyl, tert-butyl, isopentyl, neopentyl, tert-pentyl, and isohexyl; cycloalkyl groups having 3 to 18 carbon atoms such as cyclopropyl, cyclobutyl, cyclopentyl, and cyclohexyl; hydroxyl; straight-chain or branched alkoxyl groups having 1 to 18 carbon atoms such as methoxy, ethoxy, propoxy, isopropoxy, butoxy, isobutoxy, sec-butoxy, tert-butoxy, hexyloxy, decyloxy, and dodecyloxy; straight-chain or branched alkylcarbonyl groups having 2 to 18 carbon atoms such as acetyl, propionyl, butanoyl, 2-methylpropionyl, heptanoyl, 2-methylbutanoyl, 3-methylbutanoyl, octanoyl, decanoyl, dodecanoyl, and octadecanoyl; arylcarbonyl groups having 7 to 11 carbon atoms such as benzoyl, and naphthoyl; straight-chain or branched alkoxycarbonyl groups having 2 to 19 carbon atoms such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, octyloxycarbonyl, tetradecyloxycarbonyl, and octadecyloxycarbonyl; aryloxycarbonyl groups having 7 to 11 carbon atoms such as phenoxycarbonyl, and naphthoxycarbonyl; arylthiocarbonyl groups having 7 to 11 carbon atoms such as phenylthiocarbonyl, and naphthoxythiocarbonyl; straight-chain or branched acyloxy groups having 2 to 19 carbon atoms such as acetoxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy, octylcarbonyloxy, tetradecylcarbonyloxy, and octadecylcarbonyloxy; arylthio groups having 6 to 20 carbon atoms such as phenylthio, 2-methylphenylthio, 3-methylphenylthio, 4-methylphenylthio, 2-chlorophenylthio, 3-chlorophenylthio, 4-chlorophenylthio, 2-bromophenylthio, 3-bromophenylthio, 4-bromophenylthio, 2-fluorophenylthio, 3-fluorophenylthio, 4-fluorophenylthio, 2-hydroxyphenylthio, 4-hydroxyphenylthio, 2-methoxyphenylthio, 4-methoxyphenylthio, 1-naphthylthio, 2-naphthylthio, 4-[4-(phenylthio)benzoyl]phenylthio, 4-[4-(phenylthio)phenoxy]phenylthio, 4-[4-(phenylthio)phenyl]phenylthio, 4-(phenylthio)phenylthio, 4-benzoylphenylthio, 4-benzoyl-2-chlorophenylthio, 4-benzoyl-3-chlorophenylthio, 4-benzoyl-3-methylthiophenylthio, 4-benzoyl-2-methylthiophenyl, 4-(4-methylthiobenzoyl)phenylthio, 4-(2-methylthiobenzoyl)phenylthio, 4-(p-tert-butylbenzoyl)phenylthio; straight-line or branched alkylthio groups having 1 to 18 carbon atoms such as methylthio, ethylthio, propylthio, isopropylthio, butylthio, isobutylthio, sec-butylthio, tert-butylthio, pentylthio, isopentylthio, neopentylthio, tert-pentylthio, octylthio, decylthio, and dodecylthio; aryl groups having 6 to 10 carbon atoms such as phenyl, tolyl, dimethylphenyl, and naphthyl; heterocyclic groups having 4 to 20 carbon atoms such as thienyl, furanyl, pyranyl, pyrrolyl, oxazolyl, thiazolyl, pyridyl, pyrimidyl, pyrazinyl, indolyl, benzofuranyl, benzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, dibenzothienyl, xanthonyl, thioxanthonyl, and dibenzofuranyl; aryloxy groups having 6 to 10 carbon atoms such as phenoxy, and naphthyloxy; straight-chain or branched alkylsulfinyl groups having 1 to 18 carbon atoms such as methylsulfinyl, ethylsulfinyl, propylsulfinyl, isopropylsulfinyl, butylsulfinyl, isobutylsulfinyl, sec-butylsulfinyl, tert-butylsulfinyl, penylsulfinyl, isopenylsulfinyl, neopentylsulfinyl, tert-penylsulfinyl, and octylsulfinyl; arylsulfinyl groups having 6 to 10 carbon atoms such as phenylsulfinyl, tolylsulfinyl, and naphthylsulfinyl; straight-chain or branched alkylsulfonyl groups having 1 to 18 carbon atoms such as methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, tert-pentylsulfonyl, and octylsulfonyl; arylsulfonyl groups having 6 to 10 carbon atoms such as phenylsulfonyl, tolylsulfonyl (tosyl group), and naphthylsulfonyl; alkyleneoxy groups represented by the general formula (4),

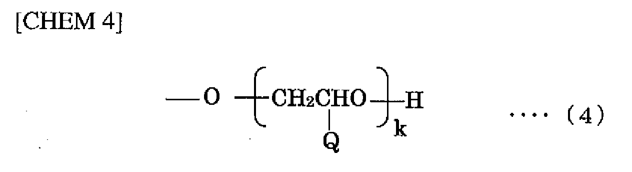

wherein Q denotes a hydrogen atom or a methyl group, and k denotes an integer of 1 to 5; a unsubstituted amino group and amino groups mono- or disubstituted by alkyl groups having 1 to 5 carbon atoms and/or aryl groups having 6 to 10 carbon atoms (Concrete examples of alkyl groups having 1 to 5 carbon atoms include straight-chain alkyl groups such as methyl, ethyl, propyl, butyl, and pentyl; branched alkyl groups such as isopropyl, isobutyl, sec-butyl, tert-butyl, isopentyl, neopentyl, and tert-pentyl; and cycloalkyl groups such as cyclopropyl, cyclobutyl, and cyclopentyl. Concrete examples of aryl groups having 6 to 10 carbon atoms include phenyl, and naphthyl); cyano; nitro; halogens such as fluorine, chlorine, bromine, and iodine.

More specifically, examples of those aryl compound (A) include benzene and benzene derivatives such as toluene, ethylbenzene, cumene, tert-butylbenzene, xylene, dodecylbenzene, nitrobenzene, benzonitrile, phenol, chlorobenzene, bromobenzene, fluorobenzene, anisole, and ethoxybenzene; naphthalene and naphthalene derivatives such as 1-methylnaphthalene, 2-methylnaphthalene, 1,2'-binaphthyl, 1-phenylnaphthalene, 2-phenylnaphthalene, 1-methoxynaphthalene, 2-ethoxynaphthalene, 1-naphthol, and 2-naphthol; anthracene and anthracene derivatives such as 9,10-dimethoxyanthracene, 2-ethyl-9,10-dimethoxyanthracene, 2-tert-butyl-9,10-dimethoxyanthracene, 2,3-dimethyl-9,10-dimethoxyanthracene, 9-methoxy-10-methylanthracene, 9,10-diethoxyanthracene, 2-ethyl-9,10-diethoxyanthracene, 2-tert-butyl-9,10-diethoxyanthracene, 2,3-dimethyl-9,10-diethoxyanthracene, 9-ethoxy-10-methylanthuacene, 9,10-dibutoxyanthracene, 2-ethyl-9,10-dibutoxyanthracene, 2-tert-butyl-9,10-dibutoxyanthracene, 2,3-dimethyl-9,10-dibutoxyanthracene, 9-butoxy-10-methylanthracene, 9,10-dipropoxyanthracene, 9,10-dibenzyloxyanthracene, diphenylanthracene, 9-methoxyanthracene, 9-ethoxyanthracene, 9-methylanthracene, 9-bromoanthracene, 9-methylthioanthracene, and 9-ethylthioanthracene; phenanthrene; naphthacene; pyrene; biphenyl; biphenylene; aryl ether derivatives such as benzyl phenyl ether, diphenyl ether, and 4-phenoxyphenol; arylsulfone derivatives such as methyl phenyl sulfone, and diphenyl sulfone; acetophenone and acetophenone derivatives such as acetylacetophenone, 2-phenylacetophenone; benzophenone and benzophenone derivatives such as 4-methylbenzophenone; sulfide derivatives such as thioanisole, ethylthiobenzene, benzyl phenyl sulfide, phenacyl phenyl sulfide, diphenyl sulfide, (2-methylphenyl)phenyl sulfide, (4-methylphenyl)phenyl sulfide, 2,2'-ditolyl sulfide, 2,3'-ditolyl sulfide, 4,4'-ditolyl sulfide, (2-phenylthio)naphthalene, 9-phenylthioanthracene, (3-chlorophenyl)phenyl sulfide, (4-chlorophenyl)phenyl sulfide, 3,3'-dichlorodiphenyl sulfide, (3-fluorophenyl)phenyl sulfide, (4-fluorophenyl)phenyl sulfide, 3,3'-difluorodiphenyl sulfide, (3-bromophenyl)phenyl sulfide, 2,2'-dibromodiphenyl sulfide, 3,3'-dibromodiphenyl sulfide, 4,4'-dichlorodiphenyl sulfide, 4,4'-dibromodiphenyl sulfide, 4,4'-difluorodiphenyl sulfide, (2-methoxyphenyl)phenyl sulfide, 4,4'-diphenylthiobenzophenone, 4,4'-diphenylthiodiphenyl ether, 4,4'-diphenylthiobiphenyl, (4-phenylthiophenyl)phenyl sulfide, (4-benzophenyl)phenyl sulfide, (2-chloro-4-benzoylphenyl)phenyl sulfide, (2-methylthio-4-benzoylphenyl)phenyl sulfide, 4-(4-tert-butylbenzoyl)phenyl phenyl sulfide, 4-(4-methylbenzoyl)phenyl phenyl sulfide; benzofuran; benzothiophene; phenothiazine; xanthene; thianthrene, phenoxathiine; dibenzothiophene; xanthone, thioxanthone and thioxanthone derivatives such as, 2-isopropylthioxanthone, 2-methoxythioxanthone, 2-methylthioxanthone, 2-ethylthioxanthone, 2-chlorothioxanthone, 2,4-diethylthioxanthone; dibenzofuran; bithiophene; and bifuran.

Among the above aryl compounds (A), those which are preferred are aryl hydrocarbons having 6 to 30 carbon atoms and which may be substituted by alkyl, hydroxyl, alkoxyl, alkylcarbonyl, arylcarbonyl, alkoxycarbonyl, aryloxycarbonyl, arylthiocarbonyl, acyloxy, arylthio, alkylthio, aryl, aryloxy, alkyleneoxy, nitro and halogen; or heterocyclic compounds having 4 to 30 carbon atoms and 1 to 2 hetero atoms selected from oxygen or sulfur, and which may be substituted by alkyl, hydroxyl, alkoxyl, alkylcarbonyl, arylcarbonyl, alkoxycarbonyl, aryloxycarbonyl, arylthiocarbonyl, acyloxy, arylthio, alkylthio, aryl, aryloxy, alkyleneoxy, nitro and halogen; and those particularly preferred are aryl hydrocarbons having 6 to 30 carbon atoms and which may be substituted by alkyl, hydroxyl, alkoxyl, arylcarbonyl, arylthio, aryl, aryloxy and halogen; or heterocyclic compounds having 4 to 30 carbon atoms and 1 to 2 hetero atoms selected from oxygen and sulfur, and which may be substituted by alkyl, hydroxyl, alkoxy, and halogen.

Among the above aryl compounds (A), those which are particularly preferred are benzene, phenol, fluorobenzene, toluene, tert-butylbenzene, anisole, benzophenone, 4-methylbenzophenone, diphenyl sulfide, (4-chlorophenyl)phenyl sulfide, 2-phenylthionaphthalene, 9-phenylthioanthracene, (4-phenylthiophenyl)phenyl sulfide, 4,4'-diphenylthiobiphenyl, (4-benzoylphenyl)phenyl sulfide, (2-chloro-4-benzoylphenyl)phenyl sulfide, 4,4'-diphenylthiobenzophenone, (4-benzoylphenyl)phenyl sulfide, 4-(4-tert-butylbenzoyl)phenyl phenyl sulfide, thianthrene, thioxanthone, phenoxathiine, dibenzothiophene, thioxanthone, 2-isopropylthioxanthone, dibenzofuran, and bithiophene. Any of these aryl compounds (A) may be employed alone, or two or more of them may be employed in combination.

In the sulfoxide compound (B) represented by formula (1), R¹ and R² are the same or different from each other, and each of them denotes a hydrocarbon group which may be substituted or a heterocycle group which may substituted. Examples of substituents on such hydrocarbon and heterocyclic groups include alkyl, hydroxyl, alkoxyl, alkylcarbonyl, arylcarbonyl, alkoxycarbonyl, aryloxycarbonyl, arylthiocarbonyl, acyloxy, arylthio, alkylthio, aryl, heterocycle, aryloxy, alkylsulfinyl, arylsulfinyl, alkylsulfonyl, arylsulfonyl, alkyleneoxy, amino, cyano, nitro and halogen, and the hydrocarbon and heterocycle groups may be substituted by one or more of the substituents. And, in the case where R¹ and R² are bonded with each other directly or via -O-, -S-, -SO-, -SO₂-, -NH-, -NR'-, -CO-, -COO-, -CONH-, an alkylene group having 1 to 3 carbon atoms or a phenylene group to form a ring structure which may be substituted, wherein R' denotes an alkyl group having 1 to 5 carbon atoms or an aryl group having 6 to 10 carbon atoms, examples of the substituents are as defined above for each of R¹ and R², and the ring structure may be substituted by one or more of them.

In the above, examples of the hydrocarbon group include aryl groups having 6 to 30 carbon atoms, alkyl groups having 1 to 30 carbon atoms, alkenyl groups having 2 to 30 carbon atoms, or alkynyl groups having 2 to 30 carbon atoms. Concrete examples of such aryl groups having 6 to 30 carbon atoms include monocyclic aryl groups such as phenyl, and condensed polycyclic aryl groups such as naphthyl, anthracenyl, phenanthrenyl, pyrenyl, chrysenyl, naphthacenyl, benzanthracenyl, anthraquinonyl, fluorenyl, and naphthoquinonyl.

Of the above hydrocarbon groups, examples of the above alkyl groups having 1 to 30 carbon atoms include straight-chain alkyl groups such as methyl, ethyl, propyl, butyl, pentyl, octyl, decyl, dodecyl, tetradecyl, hexadecyl, and octadecyl; branched alkyl groups such as isopropyl, isobutyl, sec-butyl, tert-butyl, isopentyl, neopentyl, tert-pentyl, and isohexyl; cycloalkyl groups such as cyclopropyl, cyclobutyl, cyclopentyl, and cyclohexyl: and aralkyl groups such as benzyl, naphthylmethyl, anthracenylmethyl, 1-phenylethyl, and 2-phenylethyl.

Of the above hydrocarbon groups, examples of alkenyl groups having 2 to 30 carbon atoms include straight-chain or branched groups such as vinyl, allyl, 1-propenyl, isopropenyl, 1-butenyl, 2-butenyl, 3-butenyl, 1-methyl-1-propenyl, 1-methyl-2-propenyl, 2-methyl-1-propenyl, 2-methyl-2-propenyl, 1-pentenyl, 2-pentenyl, 3-pentenyl, 4-pentenyl, 1-methyl-1-butenyl, 2-methyl-2-butenyl, 3-methyl-2-butenyl, 1,2-dimethyl-1-propenyl, 1-decenyl, 2-decenyl, 8-decenyl, 1-dodecenyl, 2-dodecenyl, 10-dodecenyl; cycloalkenyl groups including 2-cyclohexenyl, 3-cyclohexenyl; and arylalkenyl groups including styryl, and cinnamyl.

Of the above hydrocarbon groups, examples of the alkynyl group having 2 to 30 carbon atoms include those straight-chain or branched such as ethynyl, 1-propynyl, 2-propynyl, 1-butynyl, 2-butynyl, 3-butynyl, 1-methyl-2-propynyl, 1,1-dimethyl-2-propynyl, 1-pentynyl, 2-pentynyl, 3-pentynyl, 4-pentynyl, 1-decynyl, 2-decynyl, 8-decynyl, 1-dodecynyl, 2-dodecynyl, 10-dodecynyl; and arylalkynyl groups such as phenylethynyl.

Examples of the heterocyclic group having 4 to 30 carbon atoms include those having 1 to 3 hetero atoms such as oxygen, nitrogen or sulfur (they may be the same or different from one other). Concrete examples of such groups include monocyclic hetero groups such as thienyl, furanyl, pyrrolyl, oxazolyl, thiazolyl, pyridyl, pyrimidyl, and pyrazinyl; or condensed polycyclic heterocyclic groups such as indolyl, benzofuranyl, benzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, dibenzothienyl, xanthonyl, thioxanthonyl, dibenzofuranyl.

In the above, R¹ and R² may be substituted by one or more substituents, examples of which include the same substituents as those enumerated above which the aryl compound (A) may have.

In the above, R¹ and R² may be bonded with each other directly or via -O-, -S-, -SO-, -SO₂- -NH-, -NR'- (R' denotes an alkyl group having 1 to 5 carbon atoms or an aryl group having 6 to 10 carbon atoms. Concrete examples of such an alkyl group include straight-chain alkyl groups such as methyl, ethyl, propyl, butyl, pentyl and the like; branched alkyl groups such as isopropyl, isobutyl, sec-butyl, tert-butyl, isopentyl, neopentyl, and tert-pentyl; cycloalkyl groups such as cyclopropyl, cyclobutyl, cyclopentyl and the like. Concrete examples of said aryl group include phenyl, naphthyl and the like.), -CO-, -COO-, -CONH-, an alkylene group having 1 to 3 carbon atoms or a phenylene group to form a ring structure, examples of which include the following ones.

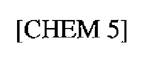

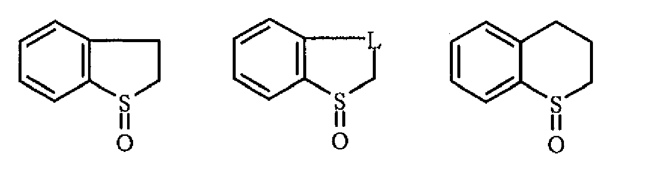

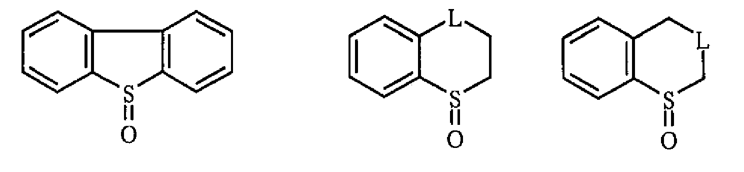

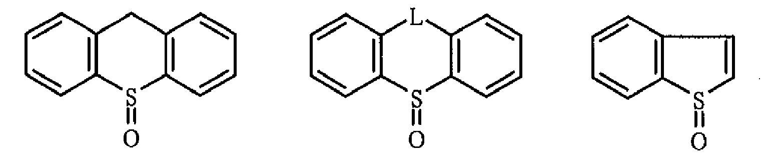

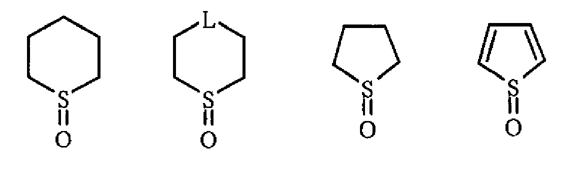

In the above, L denotes -O-, -S-, -SO-, -SO₂-, -NH-, -NR' -, -CO-, -COO-, or -CONH-, wherein R' is as defined above.

Major examples of the sulfoxide compound (B) include dialkyl sulfoxides such as dimethyl sulfoxide, methyl ethyl sulfoxide, tetramethylene sulfoxide, benzyl sulfoxide and the like; monoaryl sulfoxides such as phenacyl phenyl sulfoxide, benzyl phenyl sulfoxide, methyl phenyl sulfoxide, butyl phenyl sulfoxide, methyl-2-naphthyl sulfoxide, methyl-9-anthracenyl sulfoxide and the like; diaryl sulfoxides such as diphenyl sulfoxide, dibenzothiophene-1-oxide, (4-methylphenyl)phenyl sulfoxide, p-tolylsulfide, bis(4-methoxyphenyl)sulfoxide, (4-methylthio)phenyl phenyl sulfoxide, (4-phenylthiophenyl)phenyl sulfoxide, bis(4-hydroxyphenyl) sulfoxide, bis(4-fluorophenyl) sulfoxide, bis(4-chlorodiphenyl) sulfoxide, phenoxathiine-10-oxide, thianthrene-5-oxide, thioxanthone-10-oxide, 2-isopropylthioxanthone-10-oxide and the like.

Of the above sulfoxide compounds, preferred are those compounds in which R¹ and R² are aryl groups having 6 to 20 carbon atoms at least one of which groups may have one or more substituent groups, and particularly preferred are those compounds in which R¹ and R² are aryl groups having 6 to 20 carbon atoms both of which groups may have one or more substituent groups (diaryl sulfoxide), or those compounds in which R¹ and R² are aryl groups having 6 to 20 carbon atoms both of which groups may have one or more substituent groups and are bonded with each other directly or via -O-, -S-, -SO-, -CO-, or an alkylene group having 1 to 3 carbon atoms to form a ring structure. Of the preferred sulfoxide compound (B), particularly preferred are diphenyl sulfoxide, di(p-tolyl) sulfoxide, bis(4-methoxyphenyl) sulfoxide, bis(4-hydroxyphenyl) sulfoxide, bis(4-fluorophenyl) sulfoxide, bis(4-chlorodiphenyl) sulfoxide, phenoxathiine-10-oxide, thianthrene-5-oxide, thioxanthone-10-oxide, 2-isopropylthioxanthone-10-oxide, and dibenzothiophene-S-oxide.

Any of those sulfoxide compounds may be employed alone, or two or more of them may be employed in combination. They may be those commercially available or they may be separately synthesized for use. As desired, they may be produced in the reaction system in a preliminary process for the production of the sulfonium salt, by oxidizing a corresponding sulfide compound with a peroxide such as hydrogen peroxide.

In the formula (2) representing the fluorinated alkylfluorophosphoric acid (C) of the present invention, Rf denotes an alkyl group substituted by fluorine atoms and preferably has 1 to 8 carbon atoms. Concrete examples of such an alkyl group include straight-chain alkyl groups such as methyl, ethyl, propyl, butyl, pentyl, octyl and the like; branched alkyl groups such as isopropyl, isobutyl, sec-butyl, tert-butyl and the like; cycloalkyl groups such as cyclopropyl, cyclobutyl, cyclopentyl, cyclohexyl and the like. And the proportion of the alkyl group's hydrogen atoms which are substituted by fluorine atoms [(the number of fluorine atoms introduced into the alkyl group by the substitution)/(the number of the hydrogen atoms before the substitution) x 100] is in general not less than 80 %, where good performance is attained as an cationic polymerization initiator, and preferably not less than 90 %.

Of the above Rf, more preferable are those represented by the following formulas (5), (6) and (7).

[CHEM 6] (CF₃)_{g} CFᵢ (5)

(CF₃)_{g} CFᵢ CF₂ (6)

CF₃CF₂CF₂CF₂ (7)

In the formulas (5) and (6), g and i are integers of 0 to 3, and i + g = 3.
Particularly preferred Rf is CF₃CF₂, (CF₃)₂CF, CF₃CF₂CF₂, CF₃CF₂CF₂CF₂, or (CF₃)₂CFCF₂.

In the formula (2), "a" is an integer of 1 to 5 and indicates the number of Rf. Rf occurring "a" times may be identical with or different from one another. The number "a" of Rf is preferably 2 to 4, and particularly preferably 2 or 3.

Of the above fluorinated alkylfluorophosphoric acid (C), preferred are H[(CF₃CF₂)₂PF₄], H[(CF₃CF₂)₃PF₃], H[((CF₃)₂CF)₂PF₄], H[((CF₃)₂CF)₃PF₃], H[((CF₃)₂CF)₂PF₄], H[(CF₃CF₂CF₂)₂PF₄], H[(CF₃CF₂CF₂)₃PF₃], H[((CF₃)₂CFCF₂)₂PF₄], H[((CF₃)₂CFCF₂)₃PF₃], H[(CF₃CF₂CF₂CF₂)₂PF₄], and H[(CF₃CF₂CF₂CF₂)₃PF₃], more preferred are H[(CF₃CF₂)₃PF₃], H[(CF₃CF₂CF₂)₃PF₃], H[((CF₃)₂CFCF₂)₃PF₃], and H[((CF₃)₂CFCF₂)₂PF₄], and particularly preferred are H[(CF₃CF₂)₃PF₃], H[(CF₃CF₂CF₂)₃PF₃], H[((CF₃)₂CFCF₂)₃PF₃], and H[((CF₃)₂CFCF₂)₂PF₄].

The above fluorinated alkylfluorophosphoric acid (C) may be used as it is or in the form of a hydrate or a complex such as its diethyl ether complex, or in the form of an aqueous solution or a solution in organic acids like acetic acid or in organic solvents like diethyl ether.

The above fluorinated alkylfluorophosphoric acid (C) may be formed before or during the reaction of the aryl compound (A) with the sulfoxide compound (B), either within or outside the reaction system. Examples of the method for forming the fluorinated alkylfluorophosphoric acid (C) include a method in which fluorinated alkylphosphorane represented by the general formula (8)

[CHEM 7] (Rf)ₙ PF₅₋ₙ (8)

wherein Rf is as defined above, and n denotes an integer of 1 to 5, is let react with hydrogen fluoride; and a method in which a salt of fluorinated alkylfluorophosphoric acid (C) represented by the above formula (2) with an alkali metal, alkaline earth metal, or ammonium is let react with an inorganic acid such as sulfuric acid, phosphoric acid, or hydrochloric acid.

In the salts of the above fluorinated alkylfluorophosphoric acid (C), examples alkali metals include Li, Na, and K, alkaline earth metals including Mg and Ca, quaternary ammoniums tetrahydroammonium, tetramethylammonium, ethyltrimethylmmonium, diethyldimethylammonium, triethylmethylammonium, tetraethylammonium, trimethyl-n-propylammonium, trimethylisopropylammonium, trimethyl-n-butylammonium, trimethyl isobutylammonium, trimethyl-t-butylammonium, trimethyl-n-hexylammonium, dimethyldi-n-propylammonium, dimethyldiisopropylammonium, dimethyl-n-propylisopropylammonium, methyltri-n-propyl ammonium, and methyltriisopropylammonium.

Examples of method for letting fluorinated alkylfluorophosphorane of the above formula (8) react with hydrogen fluoride include a method in which fluorinated alkylfluorophosphorane is gradually added to nonreactive solvent such as diethyl ether, generally at 0 to 30 °C with cooling, and into this is introduced an equivalent amount of hydrogen fluoride, either in the gaseous form, generally at 0 to 30 °C, produced by warming it on warmed water or dropwise in the liquid form which is cooled at about 0 to 10 °C. Though it is possible to let fluorinated alkylfluorophosphorane react with aqueous solution of hydrogen fluoride, this is not preferred, for it would require an increased amount of dehydrating agent be employed in the reaction of the aryl compound (A) with the sulfoxide compound (B).
The molar ratio of fluorinated alkylfluorophosphorane to hydrogen fluoride is generally 1 to (0.8 to 1.2), and preferably 1 to 1.

An example of the method for letting one of the above salts of fluorinated alkylfluorophosphoric acid (C) with an alkali metal, an alkaline earth metal or an ammonium react with an inorganic acid, e.g., sulfuric acid, is a method in which the salt is first dissolved or dispersed in an organic acid such as acetic acid, or an organic acid anhydride such as acetic anhydride, or a polar organic solvent such as acetonitrile, or a mixture of them, and sulfuric acid then is added dropwise to let the reaction proceed. Thought water may be used as the solvent, to employ it in a large amount is not preferable, for it would require an increased amount of dehydrating agent be employed in the reaction of the aryl compound (A) and the sulfoxide compound (B).

While the amount of the above salts of fluorinated alkylfluorophosphoric acid (C) with an alkali metal, an alkaline earth metal or an ammonium and that of the inorganic acid employed may generally be the stoicheiometric amount, good results will also be achieved with a varying amount of the inorganic acid in the range of from 0.5 to 4 folds of the stoicheiometric amount. For example, while the stoicheiometric amount of sulfuric acid is 0.5 mole relative to 1 mole of the salt in the case, for example, of the reaction between K[(CF₃CF₂)₃PF₃] and sulfuric acid, the amount of sulfuric acid may be varied in the range of from 0.5 to 4 fold of it. If less than 0.5 mole of sulfuric acid is used, failure sometimes might follow to form a necessary amount of H[(CF₃CF₂)₃PF₃]. To use more than 4.0 moles of sulfuric acid is not preferred, for it would lead sulfonation of the aryl compound (A) or the sulfoxide compound (B) and also would increase the amount of the waste liquid. The concentration of sulfuric acid is not less than 20 %, preferably not less than 50 %, and more preferably not less than 70 %.
The temperature in this reaction is 0 to 80 °C in general, and preferably 20 to 60 °C.

Of the above methods of reaction in which a salt of fluorinated alkylfluorophosphoric acid (C) with an alkali metal, an alkaline earth metal or an ammonium, is reacted with an organic acid such as sulfuric acid, phosphoric acid, hydrochloric acid or the like, a convenient and particularly preferred method is one in which the Li, Na or K salt is reacted with sulfuric acid.

Examples of the dehydrating agent (D) include inorganic oxides such as phosphorus pentoxide, phosphorus oxychloride, polyphosphoric acid, and organic anhydrides such as acetic anhydride, propionic anhydride phthalic anhydride. Any of these dehydrating may be employed alone, or two or more of them may be employed in combination. Among these, organic anhydrides such as acetic anhydride are preferred, and particularly preferred is acetic anhydride, for it is readily available.

With regard to the molar ratio between the aryl compound (A) and the sulfoxide compound (B) in the method for production according to the present invention, the amount of sulfoxide compound (B) to 1 mole of the aryl compound (A) is 0.5 to 3.0 moles in general, preferably 0.7 to 1.5 moles, more preferably 0.8 to 1.2 moles. To use less than 0.5 mole of the sulfoxide compound (B) relative to 1 mole of the aryl compound (A), would result in a lowered yield of the aimed sulfonium salt, whereas the use of more than 3.0 moles of sulfoxide compound (B) would simply increase the cost due to using it in more amount than needed.

In the method for production according to the present invention, the amount of the fluorinated alkylfluorophosphoric acid (C) employed may be 1 equivalent stoicheiometrically relative to 1 equivalent amount of the aryl compound (A) or the sulfoxide compound (B), whichever the smaller, but to employ a little excess amount is preferred in order for accelerating the rate of the reaction. Namely, the equivalent amount of the fluorinated alkylfluorophosphoric acid (C) relative to 1 equivalent amount of the aryl compound (A) or the sulfoxide compound (B), whichever the smaller, may be 1.0 to 1.5 in general, and preferably 1.0 to 1.3. To use less than 1.0 equivalent of fluorinated alkylfluorophosphoric acid (C) relative to 1 equivalent amount of the aryl compound (A) or the sulfoxide compound (B), whichever the smaller, would result in lowered yield of the sulfonium salt, whereas increasing the amount of the fluorinated alkylfluorophosphoric acid (C) beyond 1.5 moles would be nothing but increasing the cost.

The reaction in the present invention is dehydration condensation between the aryl compound (A) and the sulfoxide compound (B). As excess amount of water present in the reaction system would lower the rate of the reaction, thereby resulting in lowered yield, the reaction must be done in the presence of a dehydrating agent (D). The amount of the dehydrating agent (D) employed is 2 fold in equivalence, or a little in excess, relative to the water amount in the reaction system, which is the total amount of the water formed by the reaction between the aryl compound (A) and the sulfoxide compound (B) and the water contained in the raw materials employed in the reaction. In the case where acetic anhydride is used as the dehydrating, agent, for example, the amount of it employed may be in the range of from 1.5 to 4.5 equivalents in general, and preferably in the range of from 2.0 to 3.5 equivalents relative to the water amount in the reaction system. To use less than 1.5 equivalents of the dehydrating agent (D) would result in lowered reaction rate, thereby taking a prolonged reaction time. The use of more than 3.5 equivalents would mean the dehydrating agent being used more than required, and thus increase the cost and cause such problems of leaving increased amount of waste liquid after the reaction.

The reaction according to the present invention may be performed in the presence of a solvent. Examples of such a solvent include alcohols such as methanol, ethanol, propanol, isopropanol, butanol, isobutanol, and tert-butanol; ketones such as acetone, and methyl ethyl ketone; esters such as ethyl acetate and butyl acetate; ethers such as diethyl ether, dipropyl ether, diisopropyl ether, dibutyl ether, diisobutyl ether, di-tert-butyl ether, ethylene glycol dimethyl ether, diethylene glycol dimethyl ether, ethylene glycol diethyl ether, diethylene glycol diethyl ether, propylene glycol dimethyl ether, dipropylene glycol dimethyl ether, tetrahydrofuran, hexahydrofuran, and dioxane; chlorinated organic solvents such as chloroform and dichloromethane; organic acids such as formic acid, acetic acid, propionic acid, methanesulfonic acid, and ethanesulfonic acid; organic acid anhydrides such as acetic anhydride and propionic anhydride; organic polar solvents such as acetonitrile and nitromethane. Any of these solvents may be employed alone, or two or more of them may be employed in combination. Of these solvents, preferred are ethers, chlorinated organic solvents, organic acids, organic acid anhydrides, and organic polar solvents like nitriles, and particularly preferred are diethyl ether, dichloromethane, acetic acid, acetic anhydride, and acetonitrile.

The amount of a solvent employed is 0 to 80 wt% in general, and preferably 20 to 60 wt%, of the total amount of the aryl compound (A), the sulfoxide compound (B), the fluorinated alkylfluorophosphoric acid (C), the dehydrating agent (D) and the solvent itself.

In the method for production according to the present invention, there is no specific restriction with regard to the order in which the raw materials are added. In general, the dehydrating agent (D), and a solvent as needed, is first put in a reaction vessel, and the sulfoxide compound (B) then is added and mixed to dissolve, which is followed by gradual addition of the fluorinated alkylfluorophosphoric acid (C), and then the aryl compound (A) is added.
In the case where the fluorinated alkylfluorophosphoric acid (A) is formed in the reaction system, one may, for example, either put a solvent first in a reaction vessel and let the reaction take place in it to form the fluorinated alkylfluorophosphoric acid (C), add, then, the sulfoxide compound (B) to the solution thus obtained and mix it to dissolve, and then add the dehydrating agent (D) and the aryl compound (A), or he may put the dehydrating agent (D), and a solvent as needed, in the reaction vessel, and, following addition of the aryl compound (A) and the sulfoxide compound (B), add a raw material which is employed to produce the fluorinated alkylfluorophosphoric acid (C).

The reaction temperature according to the present invention may be -30 °C to 120 °C in general, preferably 0°C to 100 °C, and particularly preferably 10 to 80 °C. Thought it depends on the reaction temperature, concentrations at which the reaction is carried out, and the vigorousness of the stirring, the reaction time may be 0.5 to 24 hours in general, and preferably 1 to 10 hours.

While the main product obtained by the method according to the present invention is a sulfonium salt which has a single sulfonio group per molecule, a small amount of bissulfonium salt having two sulfonio groups per molecule is also produced sometimes. The latter is also a useful ingredient as an initiator of photo-induced cationic polymerization as well as a photoacid generator for lithography of resists in semiconductor production.

The method according to the present invention allows easy recovery of dehydrating agents and solvents employed such as organic acid anhydrides, acetic acid and diethyl ether and other solvents, after completion of the reaction, by distillation under ambient or reduced pressure.
The temperature at which these materials are recovered is 40 to 120 °C in general, and preferably 50 to 80 °C. Application of higher temperatures than 120 °C might cause decomposition of the aimed sulfonium salt. The dehydrating agents and solvents recovered may be used.

In the method for production according to the present invention, the way to recover the produced, aimed sulfonium salt from the reaction mixture may vary depending on the property of it. For example, that may be in which the aimed compound is separated out by pouring water into the reaction mixture or, otherwise, the reaction mixture into water, and the substance thus separated out, if it is solid, is filtered off, washed with water and then dried, or the substance thus separated out, if it is liquid, is first extracted with an organic solvent such as dichloromethane, chloroform, ethyl acetate, toluene, xylene, and ether, washed with water, and the organic phase prepared by separation is concentrated to dryness. Organic solvent extraction as described may also be applicable even when the substance separated off is solid. The sulfonium salt thus obtained may be further purified, as needed, by washing, or recrystallization, or by a combination as desired of washing and recrystallization, employing a solvent or a mixture of two or more solvents such as an alcohol like methanol, ethanol, propanol, isopropanol, butanol, isobutanol, and tert-butanol; a ketone like acetone and methyl ethyl ketone; an ester like ethyl acetate and butyl acetate; an ether like diethyl ether and tetrahydrofuran; a chlorinated solvent like dichloromethane; an aromatic hydrocarbon like toluene and xylene; and an aliphatic hydrocarbon like pentane, hexane, cyclohexane, and octane.

### EXAMPLES

The present invention is described in further detail below with reference to examples. However, it is not intended that the present invention be limited by the examples.

### EXAMPLE 1

### Preparation of [4-(phenylthio)phenyl]diphenylsulfonium tris(pentafluoroethyl)-trifluorophosphate salt

In a 100-mL reaction vessel were put 14.6 g of acetonitrile and 14.5 g (34.0 mmol) of tris(pentafluoroethyl)difluorophosphorane and mixed well, and 0.68 g (34.0 mmol) of hydrogen fluoride was added dropwise to this at 0 to 5 °C. To this was added dropwise, at 10 to 20 °C, a solution of 6.2 g (30.7 mmol) of diphenyl sulfoxide, 5.7 g (30.5 mmol) of diphenyl sulfide, and 9.7 g (95.0 mmol) of acetic anhydride dissolved in advance in 10.3 g of acetonitrile, and the mixture was stirred for 30 minutes. Then, following reaction for 7 hours at 40 °C, the reaction mixture was cooled to room temperature, and to this was added 30 g of dichloromethane and mixed. This solution was washed with 60 g of water, and the organic phase was further washed three times with 20 g of water. Evaporation of dichloromethane then gave 25.9 g of pale yellow solid.
The solid thus obtained was found to contain the aimed [4-(phenylthiophenyl)]diphenylsulfonium tris(pentafluoroethyl)trifluorophosphate salt and a trace amount of the raw materials as determined by ¹⁹F-NMR, ¹H-NMR and IR spectrum. Its purity was 94 % (yield: 97 %). according to the HPLC analysis.
The aqueous phase collected after the washing process required 23.6 g of 40 % sodium hydroxide aqueous solution for neutralization.

### EXAMPLE 2

### Preparation of [4-(phenylthio)phenyl]diphenylsulfonium tris(pentafluoroethyl)-trifluorophosphate salt

In a 100-mL reaction vessel were put 18.3 g (37.8 mmol) of potassium tris(pentafluoroethyl)trifluorophosphate and 14.1 g of acetonitrile and mixed with stirring, and to this was added dropwise 3.8 g (38.7 mmol) of concentrated sulfuric acid, and the mixture was stirred for 30 minutes.
To this solution was added dropwise, at room temperature, a solution of 6.2 g (30.7 mmol) of diphenyl sulfoxide and 9.7 g (95.0 mmol) of acetic anhydride homogeneously dissolved in advance, and then 5.9 g (31.7 mmol) of diphenyl sulfide was added dropwise. Following reaction for 6 hours at 45°C, the reaction mixture was cooled to room temperature, and to this was added 30 g of dichloromethane and mixed. The solution was washed with 60 g of water, and the organic phase was further washed three times with 20 g of water. Evaporation of dichloromethane gave 24.8 g of pale yellow solid.
The solid thus obtained was confirmed to be the aimed (4-phenylthio phenyl)diphenylsulfonium tris(pentafluoroethyl)trifluorophosphate salt as determined by ¹⁹F-NMR, ¹H-NMR and IR spectrum, and its purity was 96 % (yield: 95 %) according to the HPLC analysis.
The aqueous phase collected after the washing process required 23.8 g of 40 % sodium hydroxide aqueous solution for neutralization.

### EXAMPLE 3

### Preparation of [4-(phenylthio)phenyl]diphenylsulfonium tris(pentafluoroethyl)-trifluorophosphate salt

In a 100-mL reaction vessel were put 5.9 g (31.7 mmol) of diphenyl sulfide, 6.2 g (30.7 mmol) of diphenyl sulfoxide, 14.1 g of acetonitrile, 9.7 g (95.0 mmol) of acetic anhydride, and 18.3 g (37.8 mmol) of potassium tris(pentafluoroethyl)trifluorophosphate, and mixed with stirring for 30 minutes. To the solution thus obtained was added dropwise 3.8 g (38.7 mmol) of concentrated sulfuric acid while controlling the temperature of the solution not to rise beyond 45 °C. Following reaction for 6 hours at 45 °C, the reaction mixture was cooled to the room temperature, and to this was added 30 g of dichloromethane and mixed. The solution was washed with 60 g of water, and the organic phase was further washed three times with 20 g of water. Evaporation of dichloromethane gave 25.1 g of pale yellow solid.
The solid thus obtained was found to contain the aimed [4-(phenylthio)phenyl]diphenylsulfonium tris(pentafluoroethyl)trifluorophosphate salt and a trace amount of the raw materials as determined by ¹⁹F-NMR, ¹H-NMR and IR spectrum. Its purity was 96 % (yield: 96 %) according to the HPLC analysisₒ
To this solid were added 40 g of toluene to dissolve, and then 370 g of hexane. Crystallization took place when the solution was cooled to 10 °C. The crystals were filtered off and washed with hexane poured over them, and then dried under reduced pressure, affording 21.3 g of white crystals of the aimed compound (purity: not less than 98 %)
The aqueous phase collected after the washing process required 23.5 g of 40 % sodium hydroxide aqueous solution for neutralization.

### EXAMPLE 4

### Preparation of [4-(phenylthio)phenyl]diphenylsulfonium tris(heptafluoropropyl)-trifluorophosphate salt

The procedure of Example 3 was followed except that 24.0 g (37.8 mmol) of potassium tris(heptafluoropropyl)trifluorophosphate was substituted for 18.3 g of potassium tris(pentafluoroethyl)trifluorophosphate, and 30 g of ethyl acetate for 30 g of dichloromethane, affording 28.5 g of [4-(phenylthio)phenylldiphenylsulfonium tris(heptafluoropropyl)trifluorophosphate salt (yield: 94 %, purity: 98 %).
The aqueous phase collected after the washing process required 23.7 g of 40 % sodium hydroxide aqueous solution for neutralization.

### EXAMPLE 5

### Preparation of 4-methoxyphenyldiphenylsulfonium tris(pentafluoroethyl)-trifluorophosphate salt

The procedure of Example 3 was followed except that 3.4 g of anisole was substituted for 5.9 g of diphenyl sulfide, affording 22.0 g of 4-methoxyphenyldiphenylsulfonium tris(pentafluoroethyl)trifluorophosphate salt (yield: 93%, purity: 98 %).
The aqueous phase collected after the washing process required 23.7 g of 40 % sodium hydroxide aqueous solution for neutralization.

### EXAMPLE 6

### Preparation of 4-dibenzothienyldiphenylsulfonium tris(pentafluoroethyl)-trifluorophosphate salt

The procedure of Example 3 was followed except that 5.8 g of dibenzothiophene was substituted for 5.9 g of diphenyl sulfide, affording 24.7 g of 4-dibenzothienyldiphenylsulfonium tris(pentafluoroethyl)trifluorophosphate salt (yield: 94 %, purity: 96 %).
The aqueous phase collected after the washing process required 23.6 g of 40 % sodium hydroxide aqueous solution for neutralization.

### EXAMPLE 7

### Preparation of 7-(2-isopropyl)thioxanthonyldiphenylsulfonium tris(pentafluoroethyl)trifluorophosphate salt

The procedure of Example 3 was followed except that 16.1 of 2-isopropylthioxhantone was substituted for 5.9 g of diphenyl sulfide, along with employment of 50 g, instead of 30 g, of dichloromethane, and 25 g, instead of 20 g, of water for washing the organic phase, affording 34.2 g of 7-(2-isopropyl)thioxanthonyldiphenylsulfonium tris(pentafluoroethyl)trifluorophosphate salt (yield: 93 % as a mixture, purity 95 %).
The aqueous phase collected after the washing process required 23.6 g of 40 % sodium hydroxide aqueous solution for neutralization.

### COMPARATIVE EXAMPLE 1

### Preparation of [4-(phenylthio)phenyl]diphenylsulfonium tris(pentafluoroethyl)-trifluorophosphate salt

In a 100-mL reaction vessel were put 7.7 g (38.1 mmol) of diphenyl sulfoxide, 5.9 g (31.7 mmol) of diphenyl sulfide, and 27.3 g (284.1 mmol) of methanesulfonic acid, and homogeneously mixed. To the mixture then was added dropwise 5.0 g (49.0 mmol) of acetic anhydride. Following reaction for 5 hours at 40 to 50 °C, the reaction mixture was cooled to room temperature. This reaction solution was added dropwise to the 79.0 g (32.6 mmol) of 20 % aqueous solution of potassium tris(pentafluoroethyl)trifluorophosphate in a vessel, and was stirred well for one hour at room temperature. Brown and somewhat thick solid which precipitated was filtered off and washed 3 times with 45 mL of water, and then dried under reduced pressure, affording 23.9 g of brown solid (yield: 60 %).
The solid thus obtained was found to contain (4-phenylthiophenyl)-diphenylsulfonium tris(pentafluoroethyl)trifluorophosphate salt as a main product together with bissulfonium salt, unreacted raw materials, and a number of compounds whose chemical structures were unknown, as determined by ¹⁹F-NMR, ¹H-NMR and IR spectrum. Its purity was 65 % according to the HPLC analysis.
The aqueous phase collected after the reaction and the washing processes required 34.4 g of 40 % sodium hydroxide aqueous solution for neutralization.

### COMPARATIVE EXAMPLE 2

### Preparation of [4-(phenylthio)phenyl]diphenylsulfonium tris(pentafluoroethyl)-trifluorophosphate salt

In a 100-mL reaction vessel were put 36.8 g (375.2 mmol) of concentrated sulfuric acid and 2.05 g (10.1 mmol) of diphenyl sulfoxide to dissolve, and to this solution was added dropwise gradually and under ice-cooling 1.80 g (9.7 mmol) of diphenyl sulfide, and the mixture was stirred for one hour at room temperature.
Then, 60 g of ice was added to a solution of 4.94 g (10.2 mmol) of potassium tris(pentafluoroethyl)trifluorophosphate in 60 mL of water, and to this was added gradually, under ice-cooling, the above reaction mixture. White solid then precipitated. This was filtered off and washed 4 times with 18 g of water, and then dried under reduced pressure, affording 5.4 g of white powder.
The white powder thus obtained was found to contain no (4-phenylthiophenyl)diphenylsulfonium tris(pentafluoroethyl)trifluorophosphate salt, which was the expected compound having a single sulfonio group, but a bissulfonium compound having two sulfonio groups as the main product, together with a small amount of the raw materials and a compound whose structure was unknown, as determined by ¹⁹F-NMR, ¹H-NMR and IR spectrum. The purity of the bissulfonium compound was 85 % according to the result of HPLC analysis.
Further, according to the analysis of the filtrate separated in the above filtration process, the filtrate contained a compound formed by sulfonation of the diphenyl sulfide.
The aqueous phase collected after the reaction and the washing processes required 74.1 g of 40 % sodium hydroxide aqueous solution for neutralization.

Table 1 shows the results of Examples 1 to 7 and Comparative Examples 1 to 2, The table indicates that the method for production according to the present invention achieves high yield and purity of the aimed sulfonium salts, and leaves smaller amount of waste fluid.

[Table 1]

**Table 1**

| | Reaction product *1) | | Amount of waste fluid per 1 kg of aimed compound in reaction product *1) | | |
|---|---|---|---|---|---|
| | Yield (%) | Purity (%) | Total amount of aqueous phase collected after reaction and washing (kg) *3) | Amount of NaOH aqueous solution required to neutralize waste liquid (kg) | Sum (kg) |
| Example 1 | 97 | 94 | 6.1 | 1.0 | 7.1 |
| Example 2 | 95 | 96 | 6.2 | 1.0 | 7.2 |
| Example 3 | 96 | 96 | 6.1 | 1.0 | 7.1 |
| Example 4 | 94 | 95 | 5.3 | 0.9 | 6.2 |
| Example 5 | 93 | 96 | 6.3 | 1.0 | 7.3 |
| Example 6 | 94 | 95 | 6.2 | 1.1 | 7.3 |
| Example 7 | 93 | 95 | 5.0 | 0.7 | 5.7 |
| Comparative Example 1 | 60 | 65 | 20.0 | 2.2 | 22.2 |
| Comparative Example 2 *2) | 63 | 85 | 49.9 | 16.1 | 66.0 |

| | | | | | |
|---|---|---|---|---|---|
| * 1) Unpurified *2) Values for bissulfonium salt obtained (aimed monosulfonium salt not obtained) *3) Washing with water performed until the pH of the aqueous phase used for washing rose higher than 5. | | | | | |

### INDUSTRIAL APPLICABILITY

The present invention can be utilized with advantage as a method for producing sulfonium fluorinated alkylfluorophosphate salts, especially those carrying aryl groups, which are free of toxic elements like As and Sb, and exhibit excellent performances as cationic photoinitiators and photoacid generators, which method enables to produce the aimed compounds directly and without using a large excess of acid, and thus is cost-saving and efficient.

## Claims

1. A method for production of a salt of sulfonium having as a counter ion a fluorinated alkylfluorophosphate anion, which method comprises reacting an aryl compound Ar-H (A) having a hydrogen atom bonded to at least one of the carbon atoms thereof with a sulfoxide compound (B) represented by the formula (I), 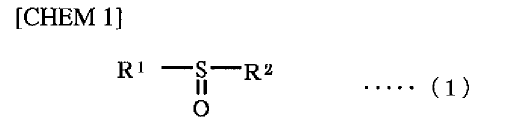 wherein R¹ and R² are the same or different from each other, and each of them denotes a hydrocarbon group which may be substituted or a heterocycle group which may be substituted, or they are bonded with each other directly or via -O-, -S-, -SO-, -SO₂-, -NH-, -NR' -, -CO-, -COO-, -CONH-, an alkylene group having 1 to 3 carbon atoms or a phenylene group to form a ring structure which may be substituted, wherein R' denotes an alkyl group having 1 to 5 carbon atoms or an aryl group having 6 to 10 carbon atoms; in the presence of a fluorinated alkylfluorophosphoric acid (C) represented by the formula (2),
[CHEM 2] H[(Rf)ₐ PF₆₋ₐ] (2)
wherein Rf denotes an alkyl group 80 % or more of whose hydrogen atoms are substituted by fluorine atoms, "a" is an integer of 1 to 5 and indicates the number of Rf, wherein Rf occurring "a" times may be identical with or different from one another; and a dehydrating agent (D) to produce the salt of sulfonium having as a counter ion a fluorinated alkylfluorophosphate anion, wherein the salt is represented by the formula (3), 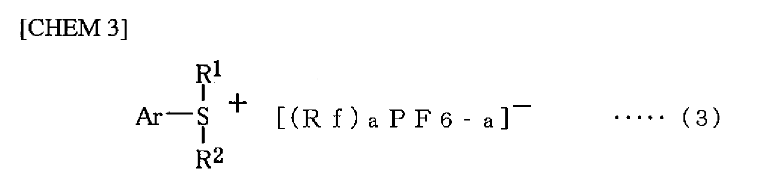 wherein R¹ and R² are as defined above, Ar denotes an aryl group derived by elimination of the hydrogen atom from the aryl compound Ar-H (A) having a hydrogen atom bonded to at least one of the carbon atoms thereof, and Rf and "a" are as defined above.

2. The method for production according to claim 1, wherein the aryl compound (A) has at least one arylthio group which may be substituted.

3. The method for production according to claim 1 or 2, wherein R¹ and R² in the formula (1) is a phenyl group which may be substituted.

4. The method for production according to one of claims 1 to 3, wherein the fluorinated alkylfluorophosphoric acid (C) is selected from the group consisting of H[(CF₃CF₂)₃PF₃], H[(CF₃CF₂CF₂)₃PF₃], H[((CF₃)₂CFCF₂)₃PF₃], and H[((CF₃)₂CF-CF₂)₂PF₄].

5. The method according to one of claims 1 to 4, wherein the dehydrating agent (D) is acetic anhydride.

6. The method according to one of claims 1 to 5, wherein the fluorinated alkylfluorophosphoric acid (C) is formed in a reaction system comprising at least any one of the aryl compound (A), the sulfoxide compound (B), the dehydrating agent (D), and a solvent by adding to the reaction system a salt of fluorinated alkylfluorophosphoric acid with an alkali metal or an alkaline earth metal, and sulfuric acid.

7. The method according to claim 6, wherein the salt of the fluorinated alkylfluorophosphoric acid is a salt of the fluorinated alkylfluorophosphoric acid with at least one alkali metal selected from the group consisting of Li, K, and Na.
